# EUROPÄISCHE PATENTSCHRIFT

(11) **EP 0 690 053 B1**
(45) Veröffentlichungstag und Bekanntmachung des Hinweises auf die Patenterteilung: **04.10.2001**
(21) Anmeldenummer: 95109689.0
(22) Anmeldetag: 22.06.1995
(51) Int. Cl.: C07D 307/28

(54) **Verfahren zur Herstellung von 2,5-Dihydrofuran**
Process for the preparation of 2,5 Dihydrofurans
Procédé pur la préparation de 2,5 Dihydrofurannes

(30) Priorität: 30.06.1994 DE 4422671
(43) Veröffentlichungstag der Anmeldung: 03.01.1996
(73) Patentinhaber: BASF Aktiengesellschaft, 67063 Ludwigshafen (DE)
(72) Erfinder: Fischer, Martin, Dr., D-67071 Ludwigshafen (DE); Fischer, Rolf, Dr., D-69121 Heidelberg (DE); John, Michael, Dr., D-67061 Ludwigshafen (DE); Pinkos, Rolf, Dr., D-67098 Bad Dürkheim (DE)

(56) Entgegenhaltungen:
- EP-A- 0 309 906
- EP-A- 0 412 366
- WO-A-91/13882
- WO-A-93/10111
- US-A- 3 812 158
- US-A- 3 932 468
- US-A- 4 231 941

## Beschreibung

Die vorliegende Erfindung betrifft ein Verfahren zur Herstellung von 2,5-Dihydrofuranen der allgemeinen Formel I 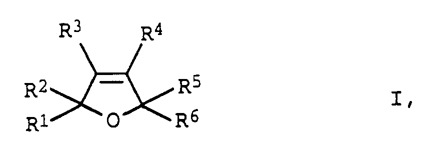 in der die Reste R¹, R², R³, R⁴, R⁵ und R⁶ gleich oder verschieden sind und für Wasserstoff oder C₁- bis C₄-Alkylgruppen stehen, durch die katalytische Umlagerung von 3,4-Epoxybutenen der allgemeinen Formel II 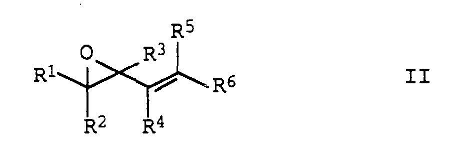 in flüssiger Phase.

2,5-Dihydrofuran ist nach US-A 3 812 158 durch die Isomerisierung von Vinyloxiran unter der Einwirkung von Quecksilbersalzen erhältlich. Abgesehen davon, daß bei diesem Verfahren 2,5-Dihydrofuran nur in maximal 33 % Ausbeute gewonnen werden kann, steht der industriellen Verwertung dieses Verfahrens die Verwendung der toxischen Quecksilbersalzkatalysatoren entgegen.

US-A 3 932 468 und US-A 3 996 248 benötigen für die Umlagerung von Vinyloxiran neben anderen Katalysatorkomponenten Brom- oder Iodwasserstoff. Nachteilig an diesen beiden Verfahren ist, daß infolge der Anwesenheit von Halogenwasserstoffen im Reaktionsmedium bei der destillativen Isolierung des 2,5-Dihydrofurans erhebliche Verluste auftreten und das erhaltene Dihydrofuran durch Halogenwasserstoff und/oder Iod verunreinigt sein kann. Außerdem treten bei der Verwendung von Halogenwasserstoffen erhebliche Korrosionsprobleme auf.

EP-A 412 366, US-A 5 034 545, WO 93/10 111, WO 91/13 882, US-A 5 082 956 und US-A 5 238 889 verwenden komplizierte Katalysatorsysteme, die u.a. Iodide enthalten. Diese Verfahren haben den Nachteil, daß die Katalysatorsysteme aufwendig und schlecht regenerierbar sind. Ferner birgt die Anwesenheit von Iodid die Gefahr, daß dieses im Form von Iod oder organischen Iodiden mit dem Produktstrom ausgetragen wird. Die Anwesenheit von Iod oder Iodiden im Produkt ist für die Weiterverarbeitung der Dihydrofurane äußerst kritisch, selbst wenn diese Verunreinigungen nur im ppm-Bereich vorliegen.

Der vorliegenden Erfindung lag somit die Aufgabe zugrunde, ein Verfahren zur Herstellung von 2,5-Dihydrofuranen durch die Umlagerung von 3,4-Epoxybutenen zu finden, das es ermöglicht, diese Umlagerung mittels eines einfachen, kostengünstigen Katalysators auf wirtschaftliche Weise durchzuführen. Insbesondere sollte ein Katalysator gefunden werden, der für die Katalyse dieser Umsetzung nicht auf den Zusatz von korrosiven Halogenwasserstoffen angewiesen ist.

Dementsprechend wurde ein Verfahren zur Herstellung von 2,5-Dihydrofuranen der allgemeinen Formel I 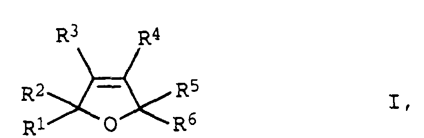 in der die Reste R¹, R², R³, R⁴, R⁵ und R⁶ gleich oder verschieden sind und für Wasserstoff oder C₁- bis C₄-Alkylgruppen stehen, durch die katalytische Umlagerung von 3,4-Epoxybutenen der allgemeinen Formel II 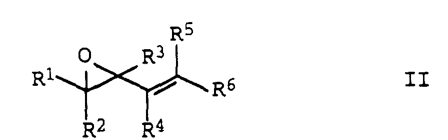 in flüssiger Phase gefunden, das dadurch gekennzeichnet ist, daß man die Umsetzung in Gegenwart eines Katalysators aus einem oder mehreren Kupfer(I)salzen durchführt.

Erfindungsgemäß werden somit als Katalysatoren für die vorgenannte Umsetzung Kupfer(I)salze verwendet. Hinsichtlich der Art der erfindungsgemäß einsetzbaren Kupfer(I)salze sind keine Beschränkungen bekannt, beispielsweise können Kupfer(I)halogenide, wie Kupfer(I)fluorid, Kupfer(I)chlorid oder Kupfer(I)bromid, Kupfer(I)sulfat, Kupfer(I)phosphat, Kupfer(I)carboxylate, wie Kupfer(I)formiat, Kupfer(I)acetat, Kupfer(I)propionat oder Kupfer(I)-2-ethylhexanoat, Kupfer(I)carbonat, Kupfer(I)cyanide oder Kupfer(I)tetrafluoroborat als Katalysatoren eingesetzt werden. Vorzugsweise werden Kupfer(I)chlorid, Kupfer(I)formiat oder Kupfer(I)acetat, insbesondere Kupfer(I)chlorid, als Katalysatoren benutzt. Selbstverständlich können auch Mischungen verschiedener Kupfer(I)salze als Katalysator verwendet werden.

Das erfindungsgemäße Verfahren wird vorzugsweise in flüssiger Phase, in An- oder Abwesenheit eines organischen Lösungsmittels durchgeführt. Soweit die verwendeten Kupfer(I)salze eine gewisse Löslichkeit im Reaktionsmedium, also dem Epoxybuten II und dem daraus gebildeten 2,5-Dihydrofuran I haben, kann der Zusatz eines Lösungsmittels unterbleiben. Vorzugsweise wird das erfindungsgemäße Verfahren jedoch in Gegenwart eines Lösungsmittels durchgeführt. Dabei ist es im allgemeinen für die erfolgreiche Durchführung der Umsetzung keine Notwendigkeit, daß das Lösungsmittel den Kupfer(I)katalysator vollständig löst. Es können eine Vielzahl unterschiedlicher Lösungsmittel, insbesondere polare aprotische Lösungsmittel, zu diesem Zweck eingesetzt werden. Beispielsweise sind Ether, wie Tetrahydrofuran, Dioxan, Ethylenglykoldialkylether, insbesondere Ethylenglykoldi-C₁-bis C₄-alkyl-ether oder Polyethylenglykoldialkylether, Pyrrolidon, N-Alkyl-pyrrolidone, insbesondere N-C₁-bis C₁₀-Alkylpyrrolidone, wie N-Methylpyrrolidon, N-Ethylpyrrolidon, N-Butylpyrrolidon, N-Cyclo-hexylpyrrolidon oder N-Octylpyrrolidon, N,N-Dialkylformamide, wie N,N-Dimethylformamid, N,N-Diethylformamid oder N,N-Dibutylformamid oder tertiäre Amine, insbesondere Tri-(C₁-bis C₁₀-alkyl-)-amine, wie Tributylamin oder Trioctylamin, geeignete Lösungsmittel. Es können auch Gemische aus verschiedenen Lösungsmitteln verwendet werden. Bevorzugt werden Lösungsmittel aus der Gruppe der Ether, der Pyrrolidone und besonders bevorzugt der Gruppe der Trialkylamine oder Gemische dieser Lösungsmittel verwendet. Die Lösungsmittelmenge kann in weiten Grenzen variiert werden. Im allgemeinen wird eine Lösungsmittelmenge von 1 bis 500 g/g Katalysator, vorzugsweise von 5 bis 250 g/g Katalysator und besonders bevorzugt von 10 bis 100 g/g Katalysator angewandt.

Das erfindungsgemäße Verfahren kann in weiten Druck- und Temperaturbereichen ausgeübt werden. Im allgemeinen wird bei einem Druck von 0,5 bis 150 bar, vorzugsweise 0,8 bis 70 bar, insbesondere von 1 bis 15 bar und bei einer Temperatur von im allgemeinen 20 bis 300°C, vorzugsweise von 50 bis 250°C und besonders bevorzugt von 80 bis 220°C gearbeitet.

Das erfindungsgemäße Verfahren kann diskontinuierlich oder bevorzugt kontinuierlich durchgeführt werden. Bei der kontinuierlichen Betriebsweise kann ein Durchsatz von im allgemeinen 0,01 bis 25, vorzugsweise von 0,1 bis 20 und besonders bevorzugt von 0,5 bis 10 kg Epoxybuten II/kg Katalysator und Stunde eingestellt werden.

Vorzugsweise wird bei der kontinuierlichen Betriebsweise des erfindungsgemäßen Verfahrens so vorgegangen, daß man das Kupfer(I)salz, gelöst oder suspendiert in einem Lösungsmittel oder Lösungsmittelgemisch, im Reaktor vorliegt und das Epoxybuten II bei der gewünschten Reaktionstemperatur dem Katalysator-Lösungsmittel-Gemisch kontinuierlich zuführt. Das sich hierbei bildende 2,5-Dihydrofuran I wird vorteilhaft, so wie es gebildet wird, kontinuierlich aus dem Reaktor abgeführt, z.B. durch Destillation. Bei dieser Vorgehensweise erweist es sich als zweckmäßig, ein Lösungsmittel oder Lösungsmittelgemisch zu verwenden, das höher siedet als das 2,5-Dihydrofuran I. Zur Beschleunigung der destillativen Abtrennung des 2,5-Dihydrofurans I aus dem Reaktionsgemisch kann z.B. bei der Umsetzung bei vermindertem Druck gearbeitet werden oder es kann das 2,5-Dihydrofuran I aus dem Reaktionsgemisch durch das Durchleiten eines Inertgasstroms, z.B. eines Stickstoffstroms, ausgetrieben werden. Eventuell bei der destillativen Abtrennung des 2,5-Dihydrofurans I mitgerissenes 3,4-Epoxybuten II kann in einer an den Reaktor angeschlossenen Destillationskolonne vom 2,5-Dihydrofuran-Produkt abgetrennt und wieder in die Umsetzung zurückgeführt werden. Ebenso ist es im Falle einer nicht vollständigen Umsetzung möglich, den Edukt-/Produktstrom in einen zweiten oder dritten Reaktor zu leiten (Kaskaden-Reaktor), um dort die Umsetzung zu Ende zu führen.

Selbstverständlich kann das erfindungsgemäße Verfahren auch in anderen Reaktoren zur Durchführung kontinuierlicher Verfahren als den geschilderten durchgeführt werden, beispielsweise in Rohrreaktoren oder Schlaufenreaktoren, und das Reaktionsprodukt 2,5-Dihydrofuran I aus dem Reaktionsaustrag außerhalb des Reaktors, beispielsweise destillativ abgetrennt werden.

Als Nebenprodukt der erfindungsgemäßen Umsetzung kann sich in geringem Umfang Crotonaldehyd bilden, der bei der destillativen Aufarbeitung der Reaktionsmischung als Wertprodukt gewonnen und anderweitig, z.B. zur Herstellung von n-Butanol, verwendet werden. Soll die Crotonaldehydbildung vermindert werden, kann dies durch die Verwendung von Trialkylaminen als Lösungsmittel oder die Verwendung Trialkylamin-haltiger Lösungsmittelgemische bewirkt werden.

Ein Zusatz von Halogenwasserstoffen ist beim erfindungsgemäßen Verfahren nicht erforderlich.

Die als Ausgangsmaterialien eingesetzten 3,4-Epoxybutene der Formel II sind auf einfache Weise, beispielsweise nach den Methoden von Kadesch (J. Am. Chem. Soc. 68, 41 (1946)) oder von WO 89/07101, erhältlich. Bevorzugt wird im erfindungsgemäßen Verfahren als 3,4-Epoxybuten II das unsubstituierte Vinyloxiran der Formel IIa 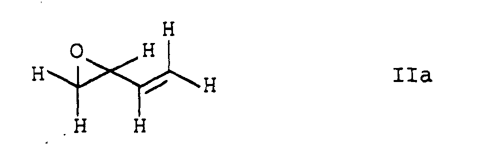 eingesetzt.

Die nach dem erfindungsgemäßen Verfahren erhältlichen 2,5-Dihydrofurane können nach üblichen Verfahren zu den entsprechenden Tetrahydrofuranen hydriert werden, welche als Lösungsmittel sowie als Monomere zur Herstellung von Polytetrahydrofuranen dienen.

### Beispiel

Zu einem Gemisch aus 5 g Kupfer(I)chlorid, 5 g Tri-n-butylamin und 80 g Triethylenglykoldimethylether in einem 250 ml Reaktionsgefäß mit aufgesetzter Kolonne wurden kontinuierlich 20 g 3,4-Epoxi-1-buten/h bei einer Sumpftemperatur von 175°C unter leichtem Stickstoffstrom (5 l/h) zudosiert. Der Produktstrom wurde über die Destillationskolonne kontinuierlich abgezogen. Das Destillat enthielt 38,4 % 2,5-Dihydrofuran und 5 % Crotonaldehyd, der Rest war Edukt.

## Patentansprüche

1. Verfahren zur Herstellung von 2,5-Dihydrofuranen der allgemeinen Formel I 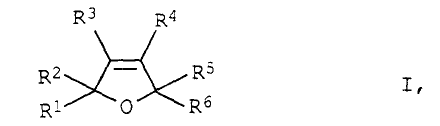 in der die Reste R¹, R², R³, R⁴, R⁵ und R⁶ gleich oder verschieden sind und für Wasserstoff oder C₁- bis C₄-Alkylgruppen stehen, durch die katalytische Umlagerung von 3,4-Epoxybutenen der allgemeinen Formel II 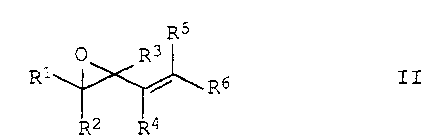 in flüssiger Phase, **dadurch gekennzeichnet, daß** man die Umsetzung in Gegenwart eines Katalysators aus einem oder mehreren Kupfer(I)salzen durchführt.

2. Verfahren nach Anspruch 1, **dadurch gekennzeichnet, daß** man als Katalysator Kupfer(I)chlorid verwendet.

3. Verfahren nach Anspruch 1 und 2, **dadurch gekennzeichnet, daß** man die Umsetzung in Gegenwart eines Lösungsmittels durchführt.

4. Verfahren nach den Ansprüchen 1 bis 3, **dadurch gekennzeichnet, daß** man die Umsetzung in Gegenwart eines Trialkylamins durchführt.

5. Verfahren nach den Ansprüchen 1 bis 4, **dadurch gekennzeichnet, daß** man die Umsetzung bei einer Temperatur von 30 bis 300°C und bei einem Druck von 0,5 bis 150 bar durchführt.

6. Verfahren nach den Ansprüchen 1 bis 5, **dadurch gekennzeichnet, daß** man Epoxybuten der Formel IIa 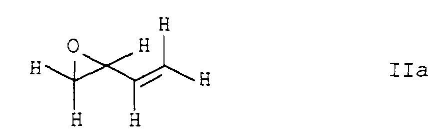 zu 2,5-Dihydrofuran der Formel Ia 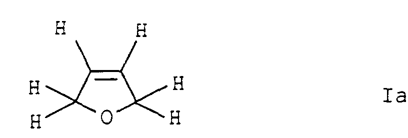 umsetzt.

## Claims

1. A process for preparing 2,5-dihydrofurans of the general formula I 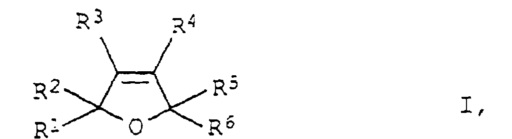 in which the radicals R¹, R², R³, R⁴, R⁵ and R⁶ are identical or different and are hydrogen or C₁ to C₄ alkyl groups, by the catalytic rearrangement of 3,4-epoxybutenes of the general formula II 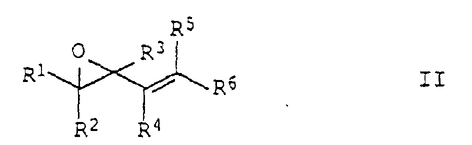 in liquid phase, which comprises conducting the reaction in the presence of a catalyst comprising one or more copper(I) salts.

2. A process as claimed in claim 1, wherein copper(I) chloride is used as catalyst.

3. A process as claimed in claim 1 and 2, wherein the reaction is conducted in the presence of a solvent.

4. A process as claimed in claims 1 to 3, wherein the reaction is conducted in the presence of a trialkylamine.

5. A process as claimed in claims 1 to 4, wherein the reaction is conducted at a temperature from 30 to 300°C and at a pressure from 0.5 to 150 bar.

6. A process as claimed in claims 1 to 5, wherein epoxybutene of the formula IIa 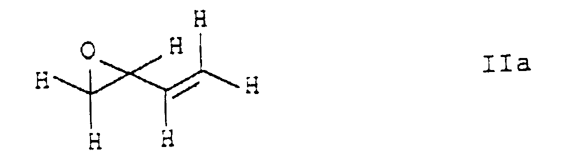 is reacted to 2,5-dihydrofuran of the formula Ia

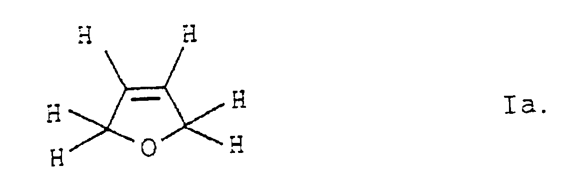

## Revendications

1. Procédé de préparation de 2,5-dihydrofurannes de formule générale I 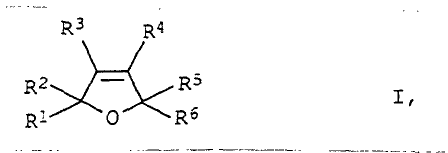 dans laquelle les radicaux R¹, R², R³, R⁴, R⁵ et R⁶ sont identiques ou différents et représentent des atomes d'hydrogène ou des groupes alkyle en C₁ à C₄, par transposition catalytique de 3,4-époxybutènes de formule générale II 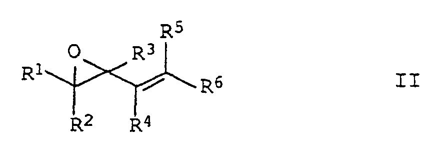 en phase liquide, **caractérisé en ce qu'**on met en oeuvre la réaction en présence d'un catalyseur à base d'un ou plusieurs sels de cuivre(I).

2. Procédé selon la revendication 1, **caractérisé en ce qu'**on utilise en tant que catalyseur du chlorure de cuivre(I).

3. Procédé selon les revendications 1 et 2, **caractérisé en ce qu'**on met en oeuvre la réaction en présence d'un solvant.

4. Procédé selon les revendications 1 à 3, **caractérisé en ce qu'**on met en oeuvre la réaction en présence d'une trialkylamine.

5. Procédé selon les revendications 1 à 4, **caractérisé en ce qu'**on met en oeuvre la réaction à une température de 30 à 300°C et sous une pression de 0,5 à 150 bar.

6. Procédé selon les revendications 1 à 5, **caractérisé en ce qu'**on fait réagir de l'époxybutène de formule IIa 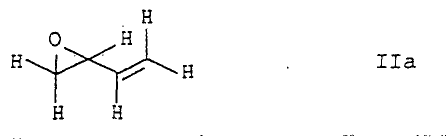 pour obtenir le 2,5-dihydrofuranne de formule Ia
